# EUROPÄISCHE PATENTSCHRIFT

(11) **EP 1 452 197 B1**
(45) Veröffentlichungstag und Bekanntmachung des Hinweises auf die Patenterteilung: **09.04.2008**
(21) Anmeldenummer: 04013434.8
(22) Anmeldetag: 07.08.1998
(51) Int. Cl.: A61M 5/50, A61M 5/32, A61M 5/20, A61M 5/28

(54) **Injektionsgerät**
Injection device
Dispositif d'injection

(30) Priorität: 24.01.1998 DE 29801168 U
(43) Veröffentlichungstag der Anmeldung: 01.09.2004
(62) Teilanmeldung aus: 98946299.9
(73) Patentinhaber: Haselmeier S.A.R.L., 1295 Mies (VD) (CH)
(72) Erfinder: Gabriel, Jochen, 70192 Stuttgart (DE)
(74) Vertreter: Raible, Tobias

(56) Entgegenhaltungen:
- EP-A- 0 577 448
- WO-A-95/19194
- US-A- 5 176 643
- US-A- 5 295 965
- US-A- 5 376 080
- US-A- 5 681 291

## Beschreibung

Die Erfindung betrifft ein Injektionsgerät mit einem Gehäuse und einem in diesem verschiebbar angeordneten Behältercontainer, sowie mit einer Nadelschutzhülse. Bevorzugtes Anwendungsgebiet ist ein Injektionsgerät zum einmaligen Gebrauch, oft auch als Einwegspritze bezeichnet.

Aus der EP-A2, 0 666 084 kennt man ein Injektionsgerät dieser Art. Es besteht aus zwei Teilen: einem wieder verwendbaren ersten Teil mit einer Energiespeicherfeder und mechanischen Elementen für die Steuerung des Ablaufs der Injektion und einem zweiten Teil mit der Injektionsflüssigkeit und der Injektionsnadel. Der zweite Teil wird nach einer Injektion weggeworfen und durch einen neuen Teil ersetzt. Er enthält auch eine Nadelschutzhülse, die nach dem Ende einer Injektion die Nadel überdeckt, um eine Kontamination durch die Nadel zu vermeiden.

Aus der US-A-5 176 643 ist ein Injektionsgerät bekannt, das ein Hauptgehäuse hat. In diesem Hauptgehäuse ist ein Spritzenkörper verschiebbar angeordnet, der mit einer Injektionsflüssigkeit gefüllt ist und der an seinem proximalen Ende mit einer Injektionsnadel versehen ist. Im Laufe eines Injektionsvorgangs verschiebt sich die Lage der Gesamtheit von Spritzenkörper und Injektionsnadel relativ zum Hauptgehäuse und entgegen der Kraft einer Rücksteilfeder. Diese verschiebt nach erfolgter Injektion die Gesamtheit von Spritzenkörper und Nadel wieder in die ursprüngliche Lage relativ zum Hauptgehäuse.

Aufgabe der Erfindung ist es, ein neues Injektionsgerät bereit zu stellen.

Nach der Erfindung wird diese Aufgabe gelöst durch den Gegenstand des Anspruchs 1. Dadurch, dass proximale und distale Stellung der Nadelschutzhülse eine Funktion der Stellung des Behältercontainers relativ zum Gehäuse sind, lassen sich diese Stellungen optimal an die Bedürfnisse vor und nach einer Injektion anpassen.

Eine sehr vorteilhafte Weiterbildung der Erfindung ist Gegenstand des Anspruchs 4. Man kann so nach einer Injektion die Nadelschutzhülse in einer gewünschten proximalen Endstellung relativ zum Gehäuse verrasten, dass sie z.B. die Injektionsnadel überdeckt und eine Kontamination durch diese sicher verhindert.

Weitere Einzelheiten und vorteilhafte Weiterbildungen der Erfindung ergeben sich aus dem im Folgenden beschriebenen und in der Zeichnung dargestellten, in keiner Weise als Einschränkung der Erfindung zu verstehenden Ausführungsbeispiel, sowie aus den Unteransprüchen. Es zeigt:
- Fig. 1: eine Darstellung eines erfindungsgemäßen Injektionsgeräts im Längsschnitt und in seiner gespannten Stellung, also der Stellung vor einer Injektion und in vergrößertem Maßstab; in der Realität hat das in Fig. 1 dargestellte Gerät z.B. eine Länge von etwa 18 cm und hat etwa die Form eines überdimensionierten Füllfederhalters,
- Fig. 2: eine Draufsicht auf die in Fig. 1 weggebrochen dargestellte Stelle, gesehen in Richtung des Pfeiles II der Fig. 1,
- Fig. 3: eine stärker vergrößerte Darstellung der oberen Hälfte des Injektionsgeräts der Fig. 1, in der gespannten Stellung des Geräts, also vor einem Injektionsvorgang,
- Fig. 4: eine Darstellung analog Fig. 3, aber nach Auslösung eines Injektionsvorgangs, wobei jedoch nur die Nadel eingestochen ist, jedoch noch keine Injektion erfolgt ist,
- Fig. 5: eine Darstellung analog den Fig. 3 und 4, aber nach vollständigem Abschluß einer Injektion,
- Fig. 6 bis 8: schematische Darstellungen zur Erläuterung des sequentiellen Ablaufs einer Injektion,
- Fig. 9: eine Darstellung des proximalen Teils des Injektors vor der Abnahme der Nadelabdeckkappe, welche die Kanüle steril abdeckt,
- Fig. 10: eine raumbildliche Darstellung zum besseren Verständnis der Fig. 9,
- Fig. 11: eine raumbildliche Darstellung des proximalen Endabschnitts der Nadelschutzhülse,
- Fig. 12: eine Darstellung des proximalen Teils des Injektors bei der Abnahme der Nadelabdeckkappe,
- Fig. 13: eine raumbildliche Darstellung zum besseren Verständnis der Fig. 12,
- Fig. 14: eine Darstellung des proximalen Teils des Injektors nach dem Einstechen der Nadel in das Unterhaut-Fettgewebe des Patienten,
- Fig. 15: eine Darstellung des proximalen Teils des Injektors nach dem Herausziehen der Nadel; diese ist hierbei von der Nadelschutzhülse rundum umgeben, um zu verhindern, daß jemand durch die Nadel verletzt oder mit einer Krankheit infiziert wird,
- Fig. 16: eine Draufsicht auf eine an der Nadelschutzhülse vorgesehene Anordnung von Widerhaken,
- Fig. 17: einen Längsschnitt, gesehen längs der Linie XVII-XVII der Fig. 16,
- Fig. 18: eine schematische Darstellung der Widerhakenanordnung vor deren Wirksamwerden, und
- Fig. 19: eine schematische Darstellung der Widerhakenanordnung nach deren Wirksamwerden.

In der folgenden Beschreibung werden die Begriffe proximal und distal in der in der Medizin üblichen Weise verwendet, also: Proximal = dem Patienten zugewandt (die Seite des Injektionsgeräts mit der Injektionsnadel 18); distal = vom Patienten abgewandt.

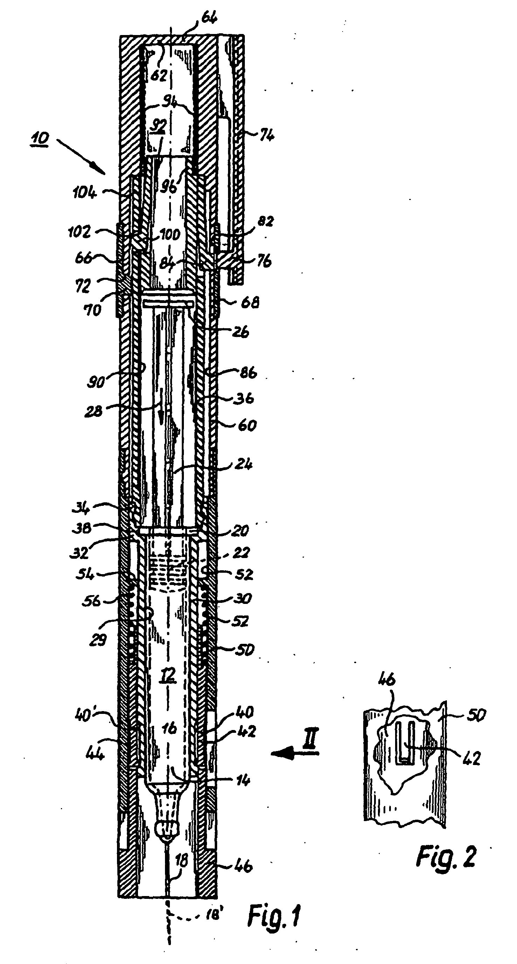

Fig. 1 zeigt die Gesamtheit eines Injektionsgeräts 10 im Längsschnitt. Beim Ausführungsbeispiel ist dies ein Injektionsgerät zum einmaligen Gebrauch, auch Autoinjektor genannt, wobei aber die Erfindung auch bei Injektionsgeräten Anwendung finden kann, die eine mehrfache Verwendung gestatten. Im Inneren des Injektionsgeräts 10 befindet sich bei dieser Ausführungsform eine Injektionsspritze 12 handelsüblicher Bauart, mit einem zylindrischenTeil 14 zur Aufnahme der Injektionsflüssigkeit 16, an dessen proximalem Ende in der üblichen Weise eine Injektionsnadel 18 befestigt ist.

Der zylindrische Teil 14 hat oben in der üblichen Weise eine Verbreiterung 20 in Form von sogenannten Spritzenflügeln. Ferner ist ein Kolben 22 vorgesehen, der mit einer Kolbenstange 24 verbunden ist, die an ihrem distalen Ende eine Druckplatte 26 hat. Drückt man in Richtung des Pfeiles 28 auf die Druckplatte 26, so wird die Flüssigkeit 16 durch die Nadel 18 ausgepreßt, wie das dem Fachmann bekannt ist.

Der zylindrische Teil 14 der handelsüblichen Spritze 12 befindet sich in der zylindrischen Ausnehmung 29 eines Behältercontainers 30, der auch als Spritzencontainer bezeichnet werden kann und der an seinem distalen Endbereich eine Schulter 32 hat, gegen deren distale Seite die Verbreiterung 20 in der dargestellten Weise anliegt. Die Schulter 32 geht über in einen kragenförmigen Abschnitt 34, der in der dargestellten Weise fest mit einem im wesentlichen zylindrisch ausgebildeten Verschiebeglied 36 verbunden ist, welches mit seinem proximalen Ende 38 die Spritzenflügel 20 einklemmt, so daß diese fest mit dem Verschiebeglied 36 und dem Behältercontainer 30 verbunden sind und die Spritze 12 zwangsweise deren Bewegungen folgt.

Der Behältercontainer 30 hat im proximalen Endbereich zwei Nuten oder Ausnehmungen 40, 40', welche einander diametral gegenüberliegen. Eine Nadelschutzhülse 46 hat zwei federnde Abschnitte 42, 44, jeder mit einem radial nach innen ragenden Vorsprung 42', 44' an seinem freien Ende. Der Vorsprung 42' ragt in die Nut 40, der Vorsprung 44' in die Nut 40'. Fig. 2 zeigt den federnden Abschnitt 42 in der Draufsicht.

Auf diese Weise ist die Nadelschutzhülse 46 zwischen einer proximalen und einer distalen Endstellung verschiebbar, deren Abstand durch die (identische) Länge der Nuten 40, 40' bestimmt ist. Verschiebt sich bei der Injektion der Behältercontainer 30 in proximaler Richtung, so ändert sich auch die Lage der Nuten 40, 40', und dadurch die proximale und die distale Endstellung der Nadelschutzhülse 46, wie das nachfolgend ausführlich beschrieben wird, d.h. beide Endstellungen verschieben sich dann in proximaler Richtung. Die Nuten 40, 40' bewirken auch eine Längsführung der Nadelschutzhülse 46.

Die Nadelschutzhülse 46 ist gleitend in der zylindrischen Innenseite 52 eines proximalen Gehäuseteils 50 verschiebbar. Von der zylindrischen Innenseite 52 ragt eine Ringschulter 54 radial nach innen. Diese dient als Widerlager für eine Druckfeder 56, welche in der dargestellten Weise die Nadelschutzhülse 46 in proximaler Richtung beaufschlagt, also in Richtung zum Patienten hin.

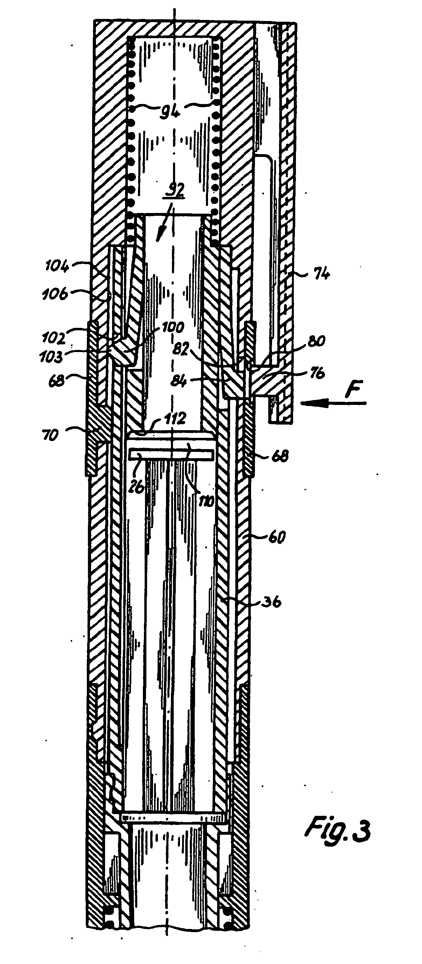

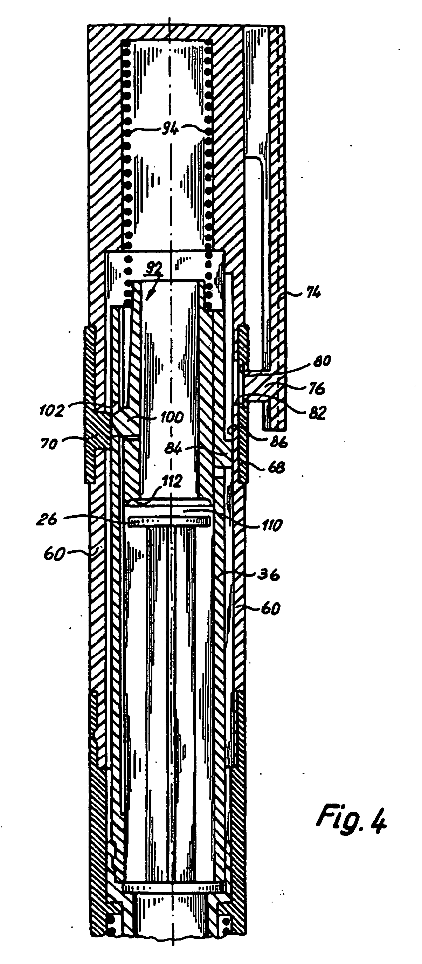

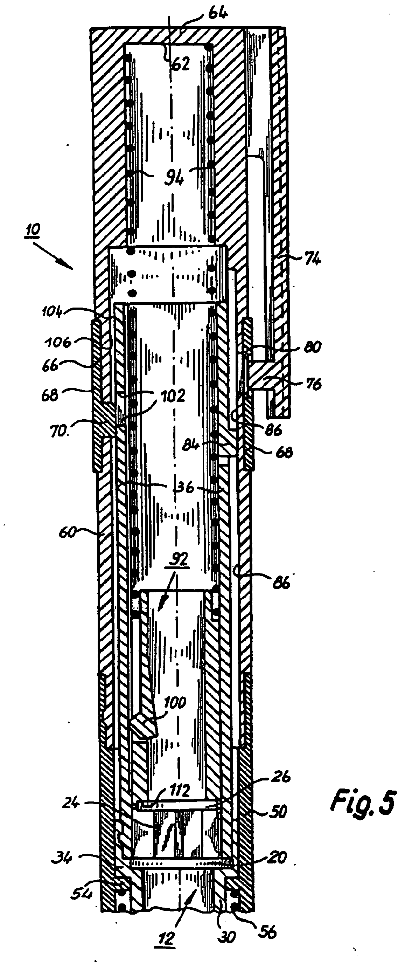

In der zylindrischen Innenseite 52 ist in der dargestellten Weise auch der kragenförmige Abschnitt 34 verschiebbar, und zwar von seiner in Fig. 1 und 3 dargestellten distalen Endstellung bis zu seiner in Fig. 4 und 5 dargestellten proximalen Endstellung, in welcher der Abschnitt 34 gegen die Ringschulter 54 anliegt.

Mit dem proximalen Gehäuseteil 50 ist ein distales Gehäuseteil 60 in der dargestellten Weise fest verbunden. Letzteres hat einen Innenraum 62, der oben, also am distalen Ende, durch eine Abschlußwand 64 verschlossen ist. Auf der Außenseite des Gehäuseteils 60 befindet sich in einer Ringnut 66 ein verdrehbares Ringteil 68, das einen Nockenabschnitt 70 hat, der durch eine Durchbrechung 72 in der dargestellten Weise in das Innere des distalen Gehäuseteils 60 hineinragt.

An der Außenseite des distalen Gehäuseteils 60 befindet sich in der dargestellten Weise ein Auslöseglied 74, das etwa die Form des Halteclips eines Füllfederhalters hat. Im Bereich seines freien (proximalen) Endes hat das Auslöseglied 74 einen radial nach innen ragenden Vorsprung 76, der dazu dient, einen Injektionsvorgang auszulösen. Bei Fig. 1 wird das verhindert durch das Ringteil 68, das sich in seiner Sperrstellung befindet und dadurch eine Bewegung des Vorsprungs 76 nach links blockiert. Die Fig. 3 bis 5 zeigen dieses Ringteil 68 in einer Drehstellung, in der es die Auslösung einer Injektion ermöglicht, weil sich dort gegenüber dem Vorsprung 76 eine Ausnehmung 80 des Ringteils 68 befindet, die dann mit einer Ausnehmung 82 des distalen Gehäuseteils 60 fluchtet.

Wie Fig. 1 zeigt, ist im gespannten Zustand in die Ausnehmung 82 ein radial nach außen federndes Rastteil 84 eingerastet, das hier einstückig mit dem Verschiebeglied 36 ausgebildet ist. Diesem Rastteil 84 ist auf der Innenseite des distalen Gehäuseteils 60 eine Längsnut 86 zugeordnet, in der sich das Rastteil 84 beim Injektionsvorgang verschiebt, vgl. die Fig. 4 und 5.

In der zylindrischen Innenseite 90 des Verschiebeglieds 36 ist ein Auspreßglied 92 gleitend verschiebbar angeordnet. Es ist von einer Druckfeder 94 in proximaler Richtung beaufschlagt, welche im gespannten Zustand (Fig. 1 und 3) die Energie speichert, die zur Durchführung eines Injektionsvorgangs erforderlich ist. Die Feder 94 ist in der dargestellten Weise mit ihrem distalen Ende am Gehäuseabschnitt 64 abgestützt, und mit ihrem proximalen Ende an einer Ringschulter 96 des Auspreßglieds 92.

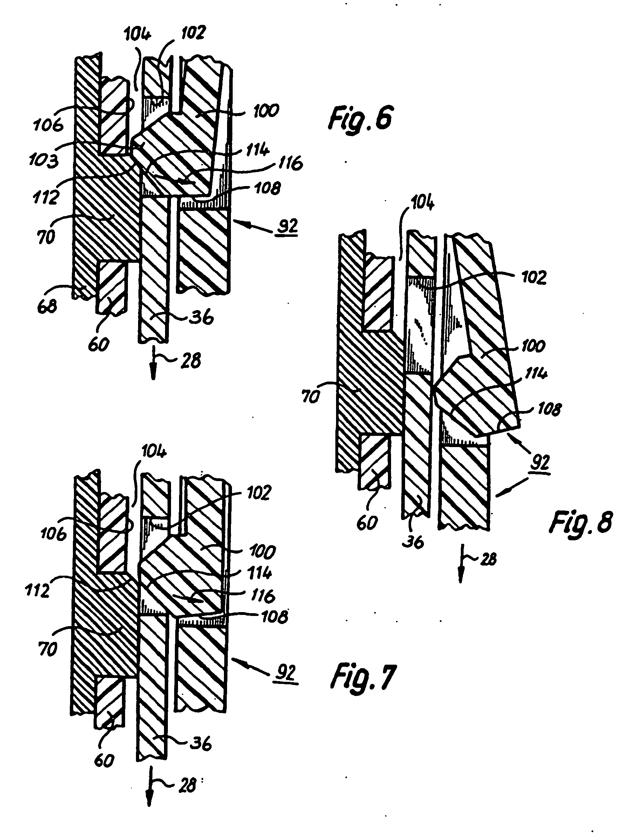

Das Auspreßglied 92 ist einstückig ausgebildet mit einem elastischen Rastglied 100, dessen Form und Funktion am besten aus den Fig. 6 bis 8 hervorgeht. Im gespannten Zustand des Injektionsgeräts 10 (Fig. 1 und 3) ragt das Rastglied 100 in eine Rastausnehmung 102 des Verschiebeglieds 36, und durch diese Ausnehmung 102 ragt es mit einem radialen Überstand 103 radial nach außen in einen radialen Freiraum oder Spalt 104 zwischen Verschiebeglied 36 und Innenseite 106 (Fig. 6 und 7) des distalen Gehäuseteils 60. Dabei stützt es sich mit einer radial verlaufenden Fläche 108 an einer entsprechenden Gegenfläche der Ausnehmung 102 ab, wie in Fig. 6 stark vergrößert dargestellt, so daß die Kraft der Feder 94 über das Rastglied 100 auf das Verschiebeglied 36 übertragen wird und dieses vor Beginn einer Injektion in proximaler Richtung beaufschlagt.

### Arbeitsweise

Zur Auslösung einer Injektion wird in Fig. 3 das Teil 74 durch eine Kraft F beaufschlagt und verschiebt dadurch das federnde Rastglied 84 des Verschiebeglieds 36 radial nach innen, so daß dieses außer Eingriff mit der Ausnehmung 82 des distalen Gehäuseteils 60 kommt.

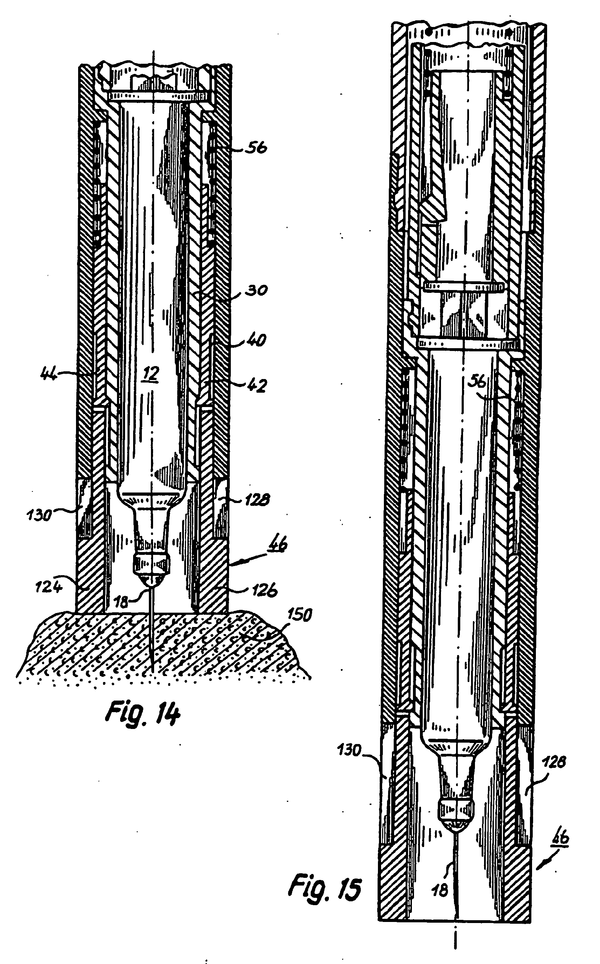

Dadurch können sich gemäß Fig. 4 unter der Wirkung der gespannten Feder 94 Auspreßglied 92 und Verschiebeglied 36 gemeinsam in proximaler Richtung verschieben, da sie durch das elastische Rastglied 100 miteinander gekoppelt sind, und die Nadel 18 wird so in die Stellung verschoben, die in Fig. 1 mit 18' bezeichnet ist, wobei sie in das Unterhaut-Fettgewebe des Patienten einsticht, vgl. Fig. 14.

Gemäß Fig. 4 bleibt hierbei zunächst ein axialer Spalt 110 zwischen dem proximalen Ende 112 des Auspreßglieds 92 und der Druckplatte 26 erhalten, da sich die Spritze 112 synchron mit dem Verschiebeglied 36 bewegt und sich folglich die Lage dieser Teile relativ zueinander nicht verändert. Die Größe des Spalts 110 hängt davon ab, wie groß die Flüssigkeitsmenge 16 in der Spritze 12 ist.

Bei Erreichen der Stellung gemäß Fig. 4 wird das elastische Rastglied 100 durch den Vorsprung 70 radial nach innen ausgelenkt, so daß es außer Eingriff mit der Ausnehmung 102 des Verschiebeglieds 36 gelangt.

Wie dies geschieht, zeigen die Fig. 6 bis 8, die eigentlich keiner Erläuterung bedürfen. Der Vorsprung 70 hat auf seiner distalen Seite eine schräge Fläche 112, der am radialen Überstand 103 eine komplementäre Schrägfläche 114 des elastischen Rastglieds 100 entspricht. Bei einer Bewegung in Richtung des Pfeils 28 gleiten die Schrägflächen 112 und 114 aufeinander und drücken das elastische Rastglied 100 in Richtung eines Pfeiles 116 radial nach innen, so daß es gemäß Fig. 7 außer Eingriff mit der zugeordneten Ausnehmung 102 des Verschiebeglieds 36 gelangt und sich gemäß Fig. 8 unter der Wirkung der Druckfeder 94 selbständig in proximaler Richtung bewegt.

Dabei drückt gemäß Fig. 5 die proximale Stirnfläche 112 des Auspreßglieds 92 gegen die Druckplatte 26 und verschiebt diese bis zum Anschlag in der handelsüblichen Spritze 12, so daß die Flüssigkeit 16 aus dieser ausgepreßt und über die Nadel 18 in den Patienten injiziert wird. Fig. 5 zeigt die Stellung, die nach Abschluß des (automatisch ablaufenden) Injektionsvorgangs erreicht wird.

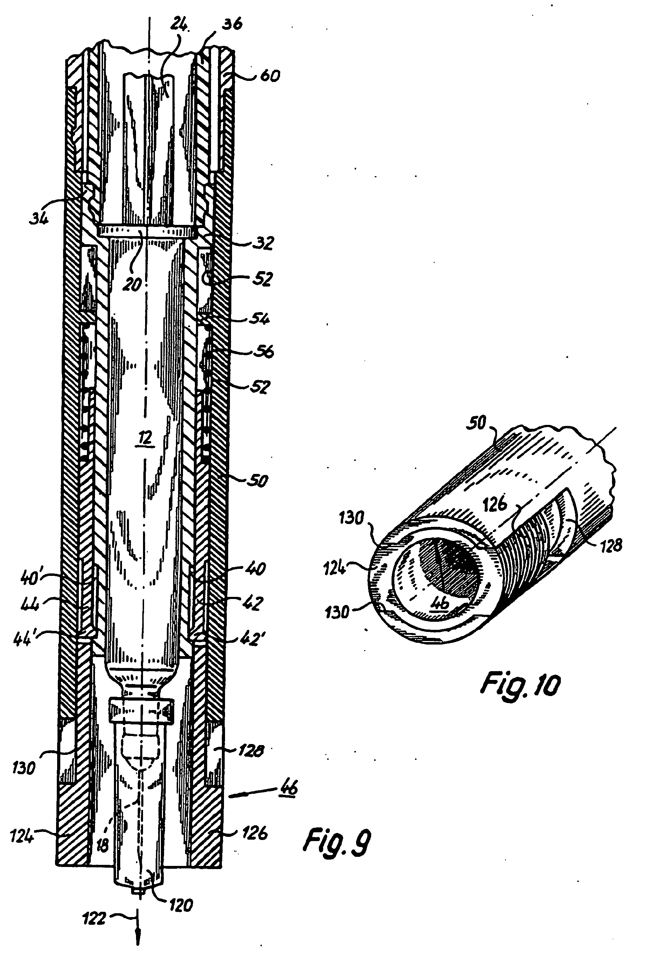

Fig. 9 stimmt mit der Darstellung gemäß Fig. 1 weitgehend überein. Sie zeigt, wie vor einer Injektion eine sterile Nadelabdeckkappe 120 in Richtung eines Pfeiles 122 abgezogen werden muß, damit die Nadel eingestochen werden kann. Im vorliegenden Fall wäre eine Abnahme der Nadelabdeckkappe 120 nur mit Hilfe einer Zange möglich.

Aus diesem Grunde hat die Nadelschutzhülse 46 zwei radiale Vorsprünge 124, 126, mit denen sie in axial verlaufende Aussparungen 128, 130 des proximalen Gehäuseteils 50 ragt und axial in diesen Aussparungen verschiebbar ist.

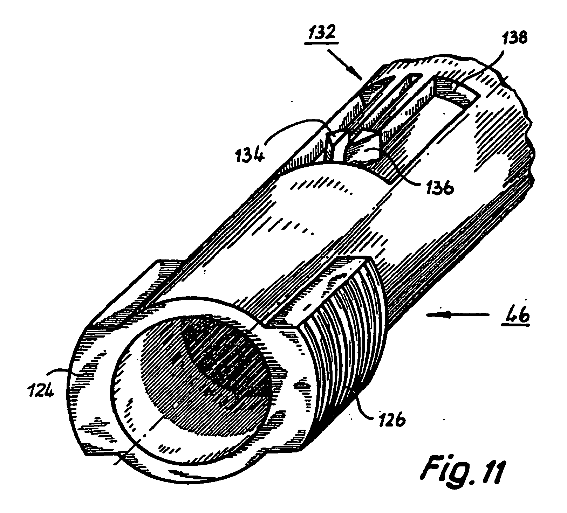

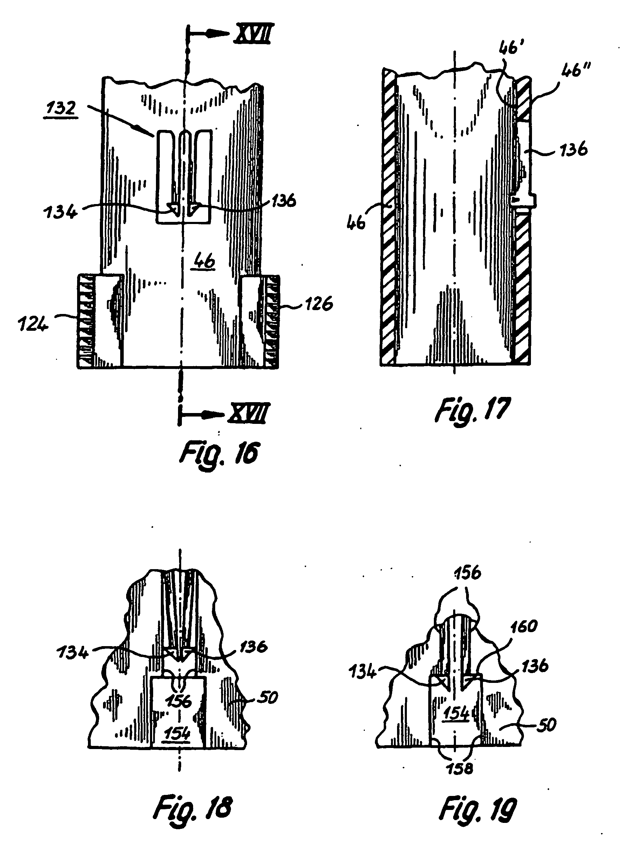

Fig. 11 zeigt in raumbildlicher Darstellung den proximalen Teil der Nadelschutzhülse 46. Diese hat auch eine Rastanordnung 132 mit zwei federnden Widerhaken 134, 136, die sich in einem Fenster 138 befinden. Die Anordnung 132 und ihre Funktion werden nachfolgend erläutert. Die Widerhaken 134, 136 ragen, wie in Fig. 17 klar dargestellt, nach innen und außen radial über den Innenumfang 46' bzw. den Außenumfang 46° der Nadelschutzhülse 46 hinaus. Der äußere Überstand dient zur Führung in einer Längsnut 154 des Gehäuseteils 50, wie in Fig. 18 und 19 dargestellt. Der innere Überstand dient dazu, bei der Montage die Widerhaken 134, 136 in Richtung zueinander auszulenken, vgl. Fig. 18.

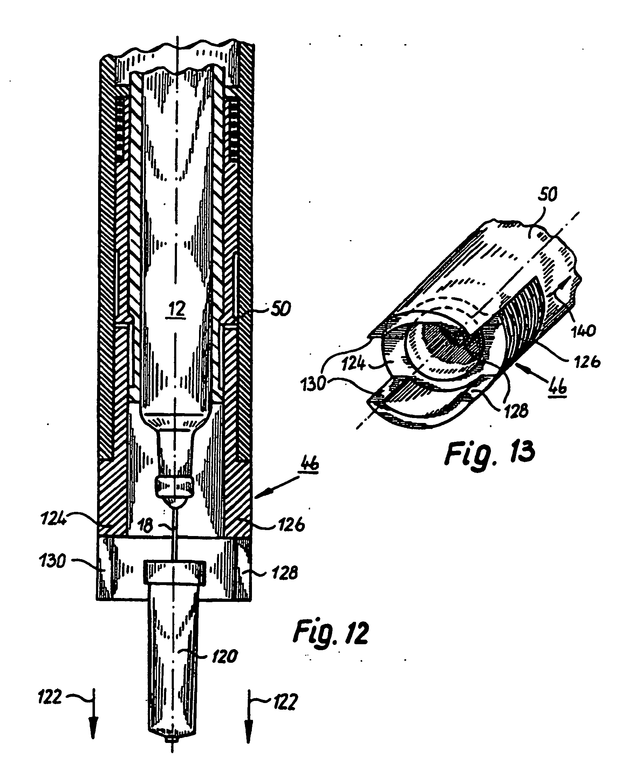

Fig. 12 und 13 zeigen, wie die Nadelschutzhülse 46 in Richtung eines Pfeiles 140 relativ zum Gehäuse 50 in distaler Richtung verschoben worden ist, so daß nun der Patient durch die Aussparungen 128, 130 hindurch die sterile Nadelabdeckkappe 120 packen und in Richtung der Pfeile 122 von der Nadel 18 abziehen kann, um eine Injektion vorzubereiten.

Fig. 14 zeigt die Nadel 18 nach dem Einstechen in das Unterhaut-Fettgewebe 150 des Patienten. Diese Stellung entspricht der Stellung, die in Fig. 4 dargestellt ist (vor dem Injizieren der Flüssigkeit) und ist identisch mit der Stellung, die in Fig. 5 dargestellt ist (nach dem Injizieren der Flüssigkeit). Der Unterschied zwischen beiden Figuren liegt in der Stellung des Kolbens 22 im Behälter 14. Dieser Kolben ist in Fig. 14 nicht dargestellt.

Die Nadelschutzhülse 46 hat bei Fig. 14 wieder die Stellung, wie sie in Fig. 9 und 10 dargestellt ist, aber ihre beiden Vorsprünge 42', 44' befinden sich nun am oberen, also distalen Ende der Nuten 40 und 40', weil sich ja der Behältercontainer 30 beim Einstechen der Nadel 18 in proximaler Richtung verschoben hat.

Dadurch hat sich also die distale Endstellung der Nadelschutzhülse 46 entsprechend verändert, und ebenso ihre proximale Endstellung, die, bezogen auf Fig. 14, weiter nach unten gewandert ist.

Wird nun gemäß Fig. 15 die Nadel 18 aus dem Unterhaut-Fettgewebe herausgezogen, so wird die Nadelschutzhülse 46 durch ihre Druckfeder 56 in ihre neue proximale Endstellung verschoben, die in Fig. 15 dargestellt ist und in der sie die Nadel 18 komplett umschließt, um einer Verletzungsgefahr vorzubeugen.

In der Stellung nach Fig. 15 wird die Nadelschutzhülse 46 dauerhaft verrastet, damit sie nicht entgegen der Kraft der Druckfeder 56 versehentlich zurückgeschoben werden kann, was dazu führen würde, daß sich jemand an der Nadel 18 verletzen oder infizieren könnte. Hierzu dienen die beiden Rasthaken 134, 136 der Vorrichtung 132, welche in Fig. 11 raumbildlich dargestellt ist. Diesen Rasthaken ist im Gehäuseteil 50 auf dessen Innenseite eine Längsnut 154 zugeordnet, die in ihrem distalen Bereich 156 schmal ist, so daß dort die Rasthaken 134, 136 zusammengepreßt werden, wie das in Fig. 18 dargestellt ist.

In der Stellung des Geräts 10 gemäß Fig. 15 gelangen, wie in Fig. 19 dargestellt, die Rasthaken 134, 136 in einen breiteren Bereich 158 am proximalen Ende der Nut 154 und rasten deshalb an der Übergangsstelle 160 ein. Dies entspricht der Stellung des Injektors nach Fig. 15, in der die Nadelschutzhülse 46 in ihrer neuen proximalen Endstellung dauerhaft verrastet ist, die damit auch zur - abschließenden - distalen Endstellung geworden ist, wenn das Injektionsgerät - nach Gebrauch - zu Abfall geworden ist.

Mit Ausnahme der Federn 56 und 94 werden die Teile des Injektionsgeräts 10 bevorzugt aus Kunststoff ausgebildet, z.B. aus ABS (Acrylnitril-Butadien-Styrol Polymerisate), PC (Polycarbonat), oder POM (Polyoxymethylen). Bevorzugte Materialien sind:
- Gehäuseteile 50, 60, Nadelschutzhülse 46, Auspreßglied 92 und Verschiebeglied 36: POM oder ABS.
- Behältercontainer 30: POM oder PC.
Die Kunststoffe werden bevorzugt einheitlich ausgewählt, um das Recycling des Injektionsgeräts zu vereinfachen.

Naturgemäß sind im Rahmen der vorliegenden Erfindung vielfache Abwandlungen und Modifikationen möglich.

## Patentansprüche

1. Injektionsgerät mit einem Gehäuse (50, 60) und einem in diesem Gehäuse zwischen einer distalen und einer proximalen Endstellung verschiebbar angeordneten Behältercontainer (30), welcher zur Aufnahme eines Behälters (14) mit Injektionsflüssigkeit ausgebildet ist,
welcher Behälter (14) mit einer Injektionsnadel (18) versehen oder verbindbar ist, so dass Bewegungen des Behältercontainers (30) in distaler sowie proximaler Richtung über den Behälter (14) auf die Injektionsnadel (18) übertragen werden und diese den Bewegungen des Behälters containers (30) folgt,
und mit einer im Bereich der Injektionsnadel (18) am proximalen Ende des Gehäuses (50, 60) angeordneten Nadelschutzhülse (46), deren Position relativ zum Gehäuse (50, 60) eine Funktion der Stellung des Behältercontainers (30) relativ zum Gehäuse (50, 60) vor einer Injektion bzw. nach einer Injektion ist.

2. Injektionsgerät nach Anspruch 1, bei welchem zwischen der Nadelschutzhülse (46) und dem Behältercontainer (30) eine mechanische Verbindung (40, 42) vorgesehen ist, welche eine Verschiebung der Nadelschutzhülse (46) relativ zum Behältercontainer (30) innerhalb vorgegebener Grenzen ermöglicht.

3. Injektionsgerät nach Anspruch 1 oder 2, bei welchem die Nadelschutzhülse (46) durch eine Feder (56) in proximaler Richtung beaufschlagt ist, welche zwischen dem Gehäuse (50) und der Nadelschutzhülse (46) wirksam ist.

4. Injektionsgerät nach Anspruch 3, bei welchem zwischen der Nadelschutzhülse (46) und dem Gehäuse (50) eine Rastanordnung (132) vorgesehen ist, welche nach Abschluss eines Injektionsvorgangs in der dann - durch die Beaufschlagung in proximaler Richtung - bewirkten proximalen Endstellung der Nadelschutzhülse (46) eine Verrastung derselben relativ zum Gehäuse (50) bewirkt.

5. Injektionsgerät nach einem oder mehreren der vorhergehenden Ansprüche, bei welchem die proximale Endstellung der Nadelschutzhülse (46) vor einem Injektionsvorgang im wesentlichen mit der distalen Endstellung der Nadelschutzhülse (46) nach einem Injektionsvorgang übereinstimmt.

6. Injektionsgerät nach Anspruch 5, bei welchem die der Nadelschutzhülse (46) in ihrer mit dem Gehäuse (50) verrasteten Stellung, welche sie nach einer Injektion einnimmt, die Injektionsnadel (18) überdeckt.

7. Injektionsgerät nach einem der vorhergehenden Ansprüche, bei welchem im Gehäuse (50, 60) ein zwischen einer distalen und einer proximalen Endstellung verschiebbar angeordnetes Verschiebeglied (36) vorgesehen ist, welches mit dem Behältercontainer (30) verbunden ist.

8. Injektionsgerät nach Anspruch 7, bei welchem eine Energiespeicherfeder (94) vorgesehen ist, um Energie für eine Injektion zu speichern, und dem Verschiebeglied (36) eine lösbare Rastvorrichtung (82, 84) zugeordnet ist, um es vor einer Injektion in einer distalen Endstellung zu verrasten, in welcher die Energiespeicherfeder (94) gespannt ist.

## Claims

1. Injection device comprising a housing (50, 60) and a container holder (30) which is arranged in this housing displaceable between a distal and a proximal end position and embodied for reception of a container (14) with injection fluid, which container (14) is provided with an injection needle (18) or capable of being connected so that movements of the container holder (30) in the distal and proximal direction are transmitted through the container (14) to the injection needle (18) and this follows the movements of the container holder (30), and a needle protection sleeve (46) which is arranged in the area of the injection needle (18) at the proximal end of the housing (50, 60) and the position of which relative to the housing (50, 60) is a function of the position of the container holder (30) relative to the housing (50, 60) prior to an injection or after an injection.

2. Injection device according to claim 1, wherein a mechanical connection (40, 42) is provided between the needle protection sleeve (46) and the container holder (30), which connection allows displacement of the needle protection sleeve (46) relative to the container holder (30) within predetermined limits.

3. Injection device according to claim 1 or 2, wherein the needle protection sleeve (46) is subject to the action of a spring (56) in the proximal direction, which spring is active between the housing (50) and the needle protection sleeve (46).

4. Injection device according to claim 3, wherein a detent arrangement (132) is provided between the needle protection sleeve (46) and the housing (50), which detent arrangement after completion of an injection operation in the proximal end position of the needle protection sleeve (46) then produced by the action in the proximal direction causes locking of the same relative to the housing (50).

5. Injection device according to one or more of the preceding claims, wherein the proximal end position of the needle protection sleeve (46) prior to an injection operation essentially coincides with the distal end position of the needle protection sleeve (46) after an injection operation.

6. Injection device according to claim 5, wherein the needle protection sleeve (46) covers the injection needle (18) in the position which it adopts after an injection and in which it is locked with the housing (50).

7. Injection device according to one of the preceding claims, wherein a displacement member (36) arranged displaceable between a distal and a proximal end position is provided in the housing (50, 60), which displacement member is connected to the container holder (30).

8. Injection device according to claim 7, wherein an energy storage spring (94) is provided in order to store energy for an injection, and the displacement member (36) has associated therewith a releasable detent device (82, 84) in order to lock it in a distal end position in which the energy storage spring (94) is tensioned prior to an injection.

## Revendications

1. Appareil d'injection comprenant un boîtier (50, 60) ; un réceptacle (30) de conteneur logé, dans ce boîtier, à coulissement entre des positions extrêmes distale et proximale, et conçu pour recevoir un conteneur (14) empli d'un liquide d'injection, lequel conteneur (14) est pourvu d'une aiguille d'injection (18) ou peut être relié à cette dernière, de telle sorte que des mouvements du réceptacle (30) du conteneur, dans les directions tant distale que proximale, soient répercutés sur ladite aiguille d'injection (18) par l'intermédiaire dudit conteneur (14), et que ladite aiguille suive les mouvements dudit réceptacle (30) du conteneur ; et une douille (46) protège-aiguille qui est située à l'extrémité proximale du boîtier (50, 60), dans la région de l'aiguille d'injection (18), et dont l'emplacement vis-à-vis dudit boîtier (50, 60) est fonction de la position occupée par le réceptacle (30) du conteneur, vis-à-vis dudit boîtier (50, 60), respectivement avant une injection et après une injection.

2. Appareil d'injection selon la revendication 1, dans lequel est prévue, entre la douille (46) protège-aiguille et le réceptacle (30) du conteneur, une solidarisation mécanique (40, 42) autorisant, dans des limites préétablies, un coulissement de ladite douille (46) protège-aiguille par rapport audit réceptacle (30) du conteneur.

3. Appareil d'injection selon la revendication 1 ou 2, dans lequel la douille (46) protège-aiguille est sollicitée, dans la direction proximale, par un ressort (56) agissant entre le boîtier (50) et ladite douille (46) protège-aiguille.

4. Appareil d'injection selon la revendication 3, dans lequel est prévu, entre la douille (46) protège-aiguille et le boîtier (50), un dispositif d'encliquetage (132) qui, à l'achèvement d'un processus d'injection, gouverne un crantage de ladite douille (46) protège-aiguille, vis-à-vis dudit boîtier (50), dans la position extrême proximale de ladite douille qui est ainsi provoquée - suite à la sollicitation dans une direction proximale-.

5. Appareil d'injection selon l'une ou plusieurs des revendications précédentes, dans lequel la position extrême proximale de la douille (46) protège-aiguille, avant un processus d'injection, coïncide pour l'essentiel avec la position extrême distale de ladite douille (46) protège-aiguille après un processus d'injection.

6. Appareil d'injection selon la revendication 5, dans lequel la douille (46) protège-aiguille coiffe l'aiguille d'injection (18) dans sa position crantée sur le boîtier (50), qu'elle prend à l'issue d'une injection.

7. Appareil d'injection selon l'une des revendications précédentes, dans lequel un organe coulissant (36), prévu dans le boîtier (50, 60), est agencé à coulissement entre des positions extrêmes distale et proximale, et est relié au réceptacle (30) du conteneur.

8. Appareil d'injection selon la revendication 7, dans lequel un ressort (94) accumulateur d'énergie est prévu pour emmagasiner de l'énergie en vue d'une injection ; et un dispositif d'encliquetage libérable (82, 84) est associé à l'organe coulissant (36) pour cranter ce dernier, préalablement à une injection, dans une position extrême distale dans laquelle ledit ressort (94) accumulateur d'énergie est bandé.
